# EUROPEAN PATENT SPECIFICATION

(11) **EP 3 528 769 B1**
(45) Date of publication and mention of the grant of the patent: **23.10.2024**
(21) Application number: 17790838.1
(22) Date of filing: 23.10.2017
(51) Int. Cl.: A61Q 5/12, A61K 8/02, A61K 8/34, A61K 8/46, A61Q 5/06, A61K 8/42, A61Q 5/10

(54) **SOLID HAIR COLOURING COMPOSITION**
FESTE HAARFÄRBEZUSAMMENSETZUNG
COMPOSITION DE COLORATION CAPILLAIRE SOLIDE

(30) Priority: 24.10.2016 GB 201617908
(43) Date of publication of application: 28.08.2019
(73) Proprietor: Cosmetic Warriors Limited, Dorset BH15 1AB (GB)
(72) Inventor: CONSTANTINE, Mark, Poole Dorset BH14 0QD (GB); CONSTANTINE, Margaret Joan, Poole Dorset BH14 0QD (GB); AMBROSEN, Helen Elizabeth, Poole Dorset BH15 0QD (GB); BIRD, Rowena Jacqueline, Christchurch Dorset BH23 4AG (GB); CAMPBELL, Daniel James, Poole Dorset BH15 1AB (GB)
(74) Representative: D Young & Co LLP
(86) International application number: PCT/GB2017/053190
(87) International publication number: WO 2018/078344

(56) References cited:
- EP-A2- 0 330 435
- WO-A1-02/47634
- WO-A1-96/09031
- GB-A- 1 231 835
- GB-A- 2 383 950
- US-A1- 2003 028 978
- US-A1- 2005 123 573
- US-A1- 2006 002 965
- DATABASE GNPD [online] MINTEL; December 2009 (2009-12-01), "Delicate soap", XP002775712, Database accession no. 1240165

## Description

### FIELD OF THE INVENTION

The present invention relates to a solid hair colouring concentrate.

### BACKGROUND TO THE INVENTION

The use of cosmetics to change the appearance of hair colour has been known for centuries. Documented evidence shows that hair dyes have been used since the ancient Egyptian period, where 'Ramesses the Great' was found to have coloured his hair using henna, whilst in ancient Greece hair was bleached and coloured using a potassium-based rinse and ointment derived from yellow flower petals.

Present day hair dying can be generally classified according to the colour durability of the hair dyes, ranging from permanent hair colourants, which are typically oxidative compositions that alter the hairs natural pigmentation, to temporary and semi-permanent hair colourants, which are non-oxidative compositions that colour the hair by depositing dye on to and within the hair fibres, but can be washed from the hair after a single or several hair washes respectively.

Permanent hair dyes possess a number of positive attributes, such as superior lightfastness and resistance to repeat washing. However their use can result in substantial damage being caused to a user's hair, due to the use of oxidative and coupling agents to lighten the melanin contained within the hair fibres, before it is coloured. These oxidative and coupling agents have been known to adversely affect the integrity of the hair's cuticle, cortex and cell membrane cortex, thereby resulting in excessive shedding or breakage of the hair fibres, split ends, dullness and reduced moisture content of the hair fibres.

Due to the disadvantages of permanent hair dyes, semi-permanent hair colourants have been growing in demand, as they are able to impart a wide-variety of colour to a user's hair, without the requirement for harmful oxidative or coupling additives to alter the hair's natural pigmentation. Furthermore consumer demand for more innovative and environmental-friendly personal care products has resulted in the commercial success of minimally packaged hair dressing products that contain little to no preservatives.

One such example of a personal hair care composition that does not require packaging and/or preservatives is referred to in WO 02/47634, whereby a composition comprising cocoa butter and henna is used to dye a user's hair.

EP 0330435 describes a solid shampoo comprising 70% to 90% detergent, preferably sodium lauryl sulphate, in solid needle form and 1% to 10% water, and optionally small amounts of various other ingredients such as conditioners, essential oils, perfumes and dyes.

US 2006/002965 describes a mixing device for mixing a pulverulent product, which is contained in a film sachet that is soluble in liquid, with said liquid and optionally at least one additional component. The aim of the disclosure is to accelerate the mixing of pulverulent products, which are packed in film sachets that are soluble in liquid, with a liquid, without the risk of creating dust.

GB 1231835 describes a solid detergent composition in tablet form that comprises 15-80% synthetic detergent and 10-50% of a water-soluble hardener.

US 2003/028978 describes a shaped body for preparing colouring compositions and for colouring keratin fibres. The shaped body contains at least one indole derivative and/or at least one indoline derivative as a dye precursor. The shaped body is placed in a composition containing water to form a colouring composition that can be applied to keratin fibres.

US 2005/123573 describes a liquid or flowable cleansing or skin treatment composition which is substantially non-aqueous and which has a continuous and discontinuous phase, where components of the discontinuous phase can chemically react with each other or with water when water is blended with the composition during consumer use.

WO 96/09031 describes a solid wash resistant hair colourant stick composition comprising a wash resistant hair dye with a physiologically acceptable solid base, and preferably an effective amount of a lathering component.

The Durance en Provence Ancian Rosa soap sold by Durance in 2009 was a soap bar that contained water, cetrimonium chloride, cetearyl alcohol, shea butter extract, olive oil and lemon extract, amongst other ingredients.

The present invention seeks to provide a solid hair colouring composition that addresses the problems of the prior art.

### SUMMARY OF THE INVENTION

In one aspect there is provided a solid hair colouring composition as set out in the appended claims. In one aspect, there is provided a solid hair colouring composition comprising (i) a hair conditioner concentrate selected from emulsifying waxes, sodium polynaphthalene sulfonate, stearamidopropyl dimethylamine (aka N-[3-(Dimethylamino)propyl] octadecanamide) and mixtures thereof; (ii) a water soluble hair colouring having a solubility of more than 20 g/L water at 25°C; (iii) an acidifying agent in an amount of from 3% to 25% by weight of the solid hair colouring composition; and (iv) a hydrocolloid, wherein the hydrocolloid is selected from cationic guar gum, behenyl trimethyl ammonium chloride, polyquaternium-81, cetrimonium bromide and mixtures thereof; wherein the solid hair colouring composition can substantially sustain its physical shape when unsupported by external means, and remains substantially solid at up to 30°C.

In one aspect there is provided a unit dose of a solid hair colouring composition, wherein the unit dose comprises (a) a solid hair colouring composition as defined herein in a predetermined amount;
(b) a stirring device for stirring a liquid; wherein the stirring device is partially embedded within the solid hair colouring composition.

In one aspect there is provided a method of preparing a hair colouring composition comprising the step of contacting water and a solid hair colouring composition as defined herein.

The present invention provides a solid hair colouring composition which by virtue of a solid nature does not require packaging and therefore offers numerous environmental advantages, such as reduction of waste product and packaging and reduced environmental impact attributable to transportation of the concentrated composition. Furthermore, because the end user contacts the solid hair colouring composition with water to form a hair colouring which is to be used shortly afterwards, the end user has a "salon style" experience of applying a high quality and freshly made colouring to their hair. Yet further, by providing a composition which does not require the high quantities of water used in the prior art compositions, a system is provided which may be free of the preservatives necessary in high water content systems.

The above advantages are achieved by providing the hair colouring in a solid form and combining it with a hair conditioner. By the provision of this combination of components, in use, the hair colouring composition may be combined with water, and in particular warm water. When contacted with water the combination of components may be stirred gently to assist in bringing water into contact with the water soluble hair colouring and combining and the hair conditioner concentrate to assist with the dispersion the components. After a short period the hair colouring is ready for use. We have found that in systems such as that referred to in WO 02/47634, whereby a composition comprising cocoa butter and henna is used to dye a user's hair, the substitution of henna with other naturally derived colourants was surprisingly found to result in colour variation and reduced colourfastness when applied to the user's hair. We have found that by providing a hair conditioner concentrate (such as an emulsifying wax) and applying this to the hair in combination with water and the heat from the warm water, the hair fibres are caused to swell and the cuticles to raise. This increases the ability of the dye to pass across the cuticle membrane and deposit within the cortex of the hair. Therefore the combination of components of the present composition together with its provision in solid form which requires solubilising before application improves the ability of the dye to permeate into the cortex and to colour the hair.

By combining the water soluble colouring with a hair conditioner concentrate we are able to provide a composition capable of dying an individual's hair utilising natural colourants, whilst simultaneously conditioning the hair fibres, so that the health of the hair is upheld during the dying process.

For ease of reference, these and further aspects of the present invention are now discussed under appropriate section headings. However, the teachings under each section are not necessarily limited to each particular section.

### DETAILED DESCRIPTION

### Composition

As discussed herein, in one aspect of the present invention, there is provided a solid hair colouring composition as defined in claim 1.

As discussed herein, the hair conditioner composition is a solid. Solid products of the present invention are compositions which can substantially sustain their physical shape when unsupported by external means, e.g. packaging etc. Thus, they are considered to be solid, solid like, in solid form or in solid-like form at room temperature. For the avoidance of doubt the solid product must remain substantially solid at up to 30°C.

By solid-like, it is understood that some materials are considered to be solid, yet over an extremely long period of time, may alter in shape, e.g. amorphous materials such as glass etc. However, they are considered to be solid-like as, for the purpose they fulfil, they are solid. The solid form of the solid compositions of the present invention means that external packaging is not required to maintain the shape of the composition.

### Hair Conditioner Concentrate

As discussed herein, in the solid hair colouring composition of the present invention contains a hair conditioner concentrate. The hair conditioner concentrate may be present in any suitable amount to provide the necessary properties of the final product. In one aspect the hair conditioner concentrate is present in an amount of at least 20 % by weight of the solid hair colouring composition. In one aspect the hair conditioner concentrate is present in an amount of at least 30 % by weight of the solid hair colouring composition. In one aspect the hair conditioner concentrate is present in an amount of at least 35 % by weight of the solid hair colouring composition. In one aspect the hair conditioner concentrate is present in an amount of at least 40 % by weight of the solid hair colouring composition. In one aspect the hair conditioner concentrate is present in an amount of at least 50 % by weight of the solid hair colouring composition.

In one aspect the hair conditioner concentrate is present in an amount of no greater than 95 % by weight of the solid hair colouring composition. In one aspect the hair conditioner concentrate is present in an amount of no greater than 90 % by weight of the solid hair colouring composition. In one aspect the hair conditioner concentrate is present in an amount of no greater than 85 % by weight of the solid hair colouring composition. In one aspect the hair conditioner concentrate is present in an amount of no greater than 80 % by weight of the solid hair colouring composition. In one aspect the hair conditioner concentrate is present in an amount of no greater than 75 % by weight of the solid hair colouring composition. In one aspect the hair conditioner concentrate is present in an amount of no greater than 70 % by weight of the solid hair colouring composition. In one aspect the hair conditioner concentrate is present in an amount of no greater than 65 % by weight of the solid hair colouring composition.

In one aspect the hair conditioner concentrate is present in an amount of from 20 to 70% by weight of the solid hair colouring composition. In one aspect the hair conditioner concentrate is present in an amount of from 25 to 70% by weight of the solid hair colouring composition. In one aspect the hair conditioner concentrate is present in an amount of from 30 to 70% by weight of the solid hair colouring composition. In one aspect the hair conditioner concentrate is present in an amount of from 40 to 70% by weight of the solid hair colouring composition. In one aspect the hair conditioner concentrate is present in an amount of from 20 to 65% by weight of the solid hair colouring composition. In one aspect the hair conditioner concentrate is present in an amount of from 25 to 65% by weight of the solid hair colouring composition. In one aspect the hair conditioner concentrate is present in an amount of from 30 to 65% by weight of the solid hair colouring composition. In one aspect the hair conditioner concentrate is present in an amount of from 40 to 65% by weight of the solid hair colouring composition. In one aspect the hair conditioner concentrate is present in an amount of from 50 to 65% by weight of the solid hair colouring composition. In one aspect the hair conditioner concentrate is present in an amount of from 20 to 60% by weight of the solid hair colouring composition. In one aspect the hair conditioner concentrate is present in an amount of from 25 to 60% by weight of the solid hair colouring composition. In one aspect the hair conditioner concentrate is present in an amount of from 30 to 60% by weight of the solid hair colouring composition. In one aspect the hair conditioner concentrate is present in an amount of from 20 to 55% by weight of the solid hair colouring composition. In one aspect the hair conditioner concentrate is present in an amount of from 25 to 55% by weight of the solid hair colouring composition. In one aspect the hair conditioner concentrate is present in an amount of from 30 to 55% by weight of the solid hair colouring composition. In one aspect the hair conditioner concentrate is present in an amount of from 20 to 50% by weight of the solid hair colouring composition. In one aspect the hair conditioner concentrate is present in an amount of from 30 to 50% by weight of the solid hair colouring composition. In one aspect the hair conditioner concentrate is present in an amount of from 20 to 45% by weight of the solid hair colouring composition. In one aspect the hair conditioner concentrate is present in an amount of from 30 to 45% by weight of the solid hair colouring composition. In one aspect the hair conditioner concentrate is present in an amount of from 20 to 40% by weight of the solid hair colouring composition. In one aspect the hair conditioner concentrate is present in an amount of from 30 to 40% by weight of the solid hair colouring composition. In one aspect the hair conditioner concentrate is present in an amount of from 35 to 40% by weight of the solid hair colouring composition.

Hair conditioner concentrates are known to those skilled in the art and are materials and compositions which condition the hair.

As the skilled person will appreciate, a hair cleansing composition differs from a hair conditioner composition or a hair conditioner concentrate. A hair cleansing composition (more commonly referred to as shampoos) typically contains surfactants that emulsify excess sebum and impurities from the hair, so that they can be washed away by the bathing water. Whilst sebum is beneficial for the condition of the hair, excess sebum gives rise to the effect of 'greasy' hair. Anionic surfactants are often used to remove excess sebum, although they can also cause the hair to become dry and brittle, without any additional conditioning. Hair cleansing compositions may also be able to provide anionic charges to the hair strands, which may volumise the hair.

In contrast, and as the skilled person will appreciate, the primary function of a hair conditioner is typically to neutralise the residual anionic charge present on the hair strands by delivering a positively charged component to the hair, thus flattening and smoothing the cuticles.

The hair conditioner concentrate is selected from emulsifying waxes, sodium polynaphthalene sulfonate, stearamidopropyl dimethylamine (aka N-[3-(dimethylamino)propyl] octadecanamide) and mixtures thereof.

The selection of emulsifying waxes as the hair conditioner concentrate is particularly preferred. The emulsifying wax may be selected from cetearyl alcohol (aka cetostearyl alcohol or cetylstearyl alcohol), stearic acid, glyceryl stearate, a combination of cetearyl alcohol and sodium lauryl sulphate (SLS), and mixtures thereof. The emulsifying wax in one aspect is a combination of cetearyl alcohol and sodium lauryl sulphate. Thus in one aspect the hair conditioner concentrate is a combination of cetearyl alcohol and sodium lauryl sulphate.

In a preferred aspect, the hair conditioner composition is a wash-out conditioner such that the user may wash the composition out of their hair after use.

When the hair conditioner concentrate is a combination of cetearyl alcohol and sodium lauryl sulphate, the cetearyl alcohol and sodium lauryl sulphate may be present in any suitable amount relative to each other. In one aspect the cetearyl alcohol and sodium lauryl sulphate are present in a ratio of from 20:1 to 2:1 by weight. In a further aspect the cetearyl alcohol and soldium lauryl sulphate are present in a ratio of from 20:1 to 4:1. In a further aspect the cetearyl alcohol and sodium lauryl sulphate are present in a ratio of from 20:1 to 5:1 by weight. In a further aspect the cetearyl alcohol and sodium lauryl sulphate are present in a ratio of from 12:1 to 8:1 by weight.

When the hair conditioner concentrate is a combination of cetearyl alcohol and sodium lauryl sulphate, the cetearyl alcohol may be present in an amount of 65 to 95 wt.% based on the combined amount of cetearyl alcohol and sodium lauryl sulphate, such as from 70 to 90 wt.% based on the combined amount of cetearyl alcohol and sodium lauryl sulphate, such as from 75 to 85 wt.% based on the combined amount of cetearyl alcohol and sodium lauryl sulphate, such as approximately 80 wt.% based on the combined amount of cetearyl alcohol and sodium lauryl sulphate.

When the hair conditioner concentrate is a combination of cetearyl alcohol and sodium lauryl sulphate, the sodium lauryl sulphate may be present in an amount of 5 to 40 wt.% based on the combined amount of cetearyl alcohol and sodium lauryl sulphate, such as from 10 to 30 wt.% based on the combined amount of cetearyl alcohol and sodium lauryl sulphate, such as from 15 to 25 wt.% based on the combined amount of cetearyl alcohol and sodium lauryl sulphate, such as approximately 20 wt.% based on the combined amount of cetearyl alcohol and sodium lauryl sulphate.

### Water Soluble Hair Colouring

As discussed here the solid hair colouring composition contains a water soluble hair colouring. The colouring can be naturally derived or it can be synthetic. Preferably it is naturally occurring. As will be appreciated by one skilled in the art a water soluble hair colouring, commonly referred to as a 'dye', can be any colouring that is completely or significantly soluble in an aqueous medium. The `Ecological and Toxicological Association of Dyes and Pigment Manufacturers' defines a dye as an "intensely coloured or fluorescent organic substances only, which impart colour to a substrate by selective absorption of light. They are typically soluble and/or go through an application process, which, at least temporarily, destroys any crystal structure by absorption, solution, and mechanical retention, or by ionic or covalent chemical bonds".

Natural colourants are known to those skilled in the art and are materials and compositions which are naturally occurring and have the ability to colour compositions. In one aspect, the natural colourant is a colourant derived from organic sources. In one aspect, the natural colourant is a colourant not derived from inorganic sources. In one aspect, the natural colourant is a colourant derived from organic, and not inorganic, sources. In one aspect, the natural colourant is an organic pigment. The terms organic and inorganic are understood by those skilled in the art. Typically, an organic material is considered to be a material containing carbon. An inorganic material is one that is not organic. The cosmetic industry has been under particular scrutiny with regards to the use of dyes and colorants that are used in cosmetic products, particularly as it has been shown that dyes derived from coal tar and colorants containing azo-compounds are potentially toxic and mutagenic. However naturally derived dyes have been known to be sensitive to light, UV radiation, temperature variation and moisture. The present invention was able to overcome this issue by formulating an anhydrous, or substantially anhydrous composition, that is mixed with water heated to a temperature of from 70-95°C, ahead of its application to the user's hair.

In one aspect, the natural colourant is a colourant that is derived from plant-based material. In one aspect, the natural colourant is a colourant that a component of a plant. In one aspect the natural colourant is selected from beetroot, chlorophyll, gardenia, blackberry, coffee, rose, caramel powder, grape, alfalfa, walnut hull, calendula, cocoa, green tea, hibiscus, kelp, olive, orange, parsley, pumpkin, spinach, spirulina, wheatgrass sources, turmeric, butterfly pea, carrot, tomato and mixtures thereof.

In one aspect the natural colourant is selected from chlorophyll, carotenoids, anthocyanins, betalains and mixtures thereof.

In one aspect the natural colourant is selected from CI Natural blue 1, CI Natural blue 2, CI Natural red 1, CI Natural red 3, CI Natural red 4, CI Natural red 6, CI Natural red 8, CI Natural red 9, CI Natural red 10, CI Natural red 11, CI Natural red 12, CI Natural red 14, CI Natural red 16, CI Natural red 18, CI Natural red 20, CI Natural red 25, CI Natural red 33, CI Natural orange 2, CI Natural orange 4, CI Natural orange 6, CI Natural yellow 2, CI Natural yellow 3, CI Natural yellow 10, CI Natural yellow 13, CI Natural yellow 16, CI Natural brown 3, CI Natural brown 7, CI Natural brown 10, CI Natural green 2, CI Natural green 3, CI Natural black 1 and mixtures thereof.

The water soluble hair colouring may be an oxidative or non-oxidative dye. Preferably, the water soluble hair colouring is a non-oxidative dye. In some embodiments, the water soluble hair colouring is a non-oxidative dye selected from Acid Yellow 23, Acid Orange 7, Acid Yellow 1, Acid Red 33, Acid Red 92, Acid Violet 43, Acid Blue 9, Acid Black 1, HC Yellow 2, HC Red 3, HC Blue 2, HC Blue 15, Basic Red 51, Basic Orange 31, Basic Brown 16, Basic Brown 17, Basic Red 76, Basic Blue 99, Basic Yellow 57, Basic Yellow 87, Basic Violet 2, 4-hydroxypropylamino-3-nitrophenol, N,N'-bis-(2-hydroxyethyl)-2-nitro-phenylenediamine, and mixtures thereof. In some embodiments, the water soluble hair colouring is selected from Acid Yellow 23, Acid Orange 7, Acid Yellow 1, Acid Red 33, Acid Red 92, Acid Violet 43, Acid Blue 9, Acid Black 1, HC Yellow 2, HC Red 3, HC Blue 2, HC Blue 15, Basic Red 51, Basic Orange 31, Basic Brown 16, Basic Brown 17, Basic Red 76, Basic Blue 99, Basic Yellow 57, Basic Yellow 87, Basic Violet 2, and mixtures thereof. In some embodiments, the water soluble colouring is a mixture of Basic Red 51 and Basic Blue 99. In some embodiments, the water soluble colouring is a mixture of Basic Blue 99, Basic Brown 16, Acid Violet 43, Basic Red 76 and Basic Yellow 57. It is noted that each of these colourings are listed using the name ascribed by the International Nomenclature of Cosmetic Ingredients (INCI).

In some embodiments, the water soluble colouring is a colouring that is suitable for use in food. In some embodiments, the water soluble colouring is a colour additive that is approved for use in food, drugs and cosmetics (FD&C colourings). In some embodiments, the water soluble colouring is selected from FD&C Blue No. 1, FD&C Blue No. 2, FD&C Green No. 3, FD&C Red No. 3, FD&C Red No. 40, FD&C Yellow No. 5, FD&C Yellow No. 6, FD&C Green No. 1, FD&C Green No. 2, FD&C Red No. 1, FD&C Red No. 2, FD&C Red No. 4, FD&C Violet No. 1, FD&C Orange No. 1, FD&C Orange No. 2, FD&C Yellow No. 1, FD&C Yellow No. 2, FD&C Yellow No. 3, FD&C Yellow No. 4, and mixtures thereof.

In some embodiments, the water soluble colouring comprises a mixture of a non-oxidative dye (preferably selected from the list provided above) with a natural colourant and/or an FD&C colouring. In some embodiments, the water soluble colouring is a mixture of a non-oxidative dye (preferably selected from the list provided above) and an FD&C colouring.

As described herein, the water soluble colouring has a solubility of more than 20 g/L water at 25°C. In one aspect a water soluble colouring has a solubility of more than 30 g/L water at 25°C. In one aspect a water soluble colouring has a solubility of more than 40 g/L water at 25°C. In one aspect a water soluble colouring has a solubility of more than 50 g/L water at 25°C. In one aspect a water soluble colouring has a solubility of more than 60 g/L water at 25°C. In one aspect a water soluble colouring has a solubility of more than 70 g/L water at 25°C. In one aspect a water soluble colouring has a solubility of more than 80 g/L water at 25°C. In one aspect a water soluble colouring has a solubility of more than 90 g/L water at 25°C. In one aspect a water soluble colouring has a solubility of more than 100 g/L water at 25°C.

The water soluble hair colouring may be present in any suitable amount to provide the necessary properties of the final product. In one aspect the water soluble hair colouring is present in an amount of at least 0.01 % by weight of the solid hair colouring composition. In one aspect the water soluble hair colouring is present in an amount of at least 0.1 % by weight of the solid hair colouring composition. In one aspect the water soluble hair colouring is present in an amount of at least 0.2 % by weight of the solid hair colouring composition. In one aspect the water soluble hair colouring is present in an amount of at least 0.5 % by weight of the solid hair colouring composition. In one aspect the water soluble hair colouring is present in an amount of at least 1 % by weight of the solid hair colouring composition. In one aspect the water soluble hair colouring is present in an amount of at least 1.5 % by weight of the solid hair colouring composition. In one aspect the water soluble hair colouring is present in an amount of at least 2 % by weight of the solid hair colouring composition.

In one aspect the water soluble hair colouring is present in an amount of no greater than 30 % by weight of the solid hair colouring composition. In one aspect the water soluble hair colouring is present in an amount of no greater than 25 % by weight of the solid hair colouring composition. In one aspect the water soluble hair colouring is present in an amount of no greater than 20 % by weight of the solid hair colouring composition. In one aspect the water soluble hair colouring is present in an amount of no greater than 15 % by weight of the solid hair colouring composition. In one aspect the water soluble hair colouring is present in an amount of no greater than 10 % by weight of the solid hair colouring composition. In one aspect the water soluble hair colouring is present in an amount of no greater than 7 % by weight of the solid hair colouring composition. In one aspect the water soluble hair colouring is present in an amount of no greater than 5 % by weight of the solid hair colouring composition. In one aspect the water soluble hair colouring is present in an amount of no greater than 4 % by weight of the solid hair colouring composition. In one aspect the water soluble hair colouring is present in an amount of no greater than 3 % by weight of the solid hair colouring composition. In one aspect the water soluble hair colouring is present in an amount of no greater than 2 % by weight of the solid hair colouring composition.

In one aspect the water soluble hair colouring is present in an amount of from 0.01 to 30% by weight of the solid hair colouring composition. In one aspect the water soluble hair colouring is present in an amount of from 0.01 to 25% by weight of the solid hair colouring composition. In one aspect the water soluble hair colouring is present in an amount of from 0.01 to 20% by weight of the solid hair colouring composition. In one aspect the water soluble hair colouring is present in an amount of from 0.01 to 15% by weight of the solid hair colouring composition. In one aspect the water soluble hair colouring is present in an amount of from 0.1 to 30% by weight of the solid hair colouring composition. In one aspect the water soluble hair colouring is present in an amount of from 0.1 to 25% by weight of the solid hair colouring composition. In one aspect the water soluble hair colouring is present in an amount of from 0.1 to 20% by weight of the solid hair colouring composition. In one aspect the water soluble hair colouring is present in an amount of from 0.1 to 15% by weight of the solid hair colouring composition. In one aspect the water soluble hair colouring is present in an amount of from 1 to 30% by weight of the solid hair colouring composition. In one aspect the water soluble hair colouring is present in an amount of from 1 to 25% by weight of the solid hair colouring composition. In one aspect the water soluble hair colouring is present in an amount of from 1 to 20% by weight of the solid hair colouring composition. In one aspect the water soluble hair colouring is present in an amount of from 1 to 15% by weight of the solid hair colouring composition. In one aspect the water soluble hair colouring is present in an amount of from 2 to 30% by weight of the solid hair colouring composition. In one aspect the water soluble hair colouring is present in an amount of from 2 to 25% by weight of the solid hair colouring composition. In one aspect the water soluble hair colouring is present in an amount of from 2 to 20% by weight of the solid hair colouring composition. In one aspect the water soluble hair colouring is present in an amount of from 2 to 15% by weight of the solid hair colouring composition. In one aspect the water soluble hair colouring is present in an amount of from 2 to 10% by weight of the solid hair colouring composition. In one aspect the water soluble hair colouring is present in an amount of from 2 to 5% by weight of the solid hair colouring composition. In one aspect the water soluble hair colouring is present in an amount of from 0.01 to 10% by weight of the solid hair colouring composition. In one aspect the water soluble hair colouring is present in an amount of from 0.01 to 5% by weight of the solid hair colouring composition. In one aspect the water soluble hair colouring is present in an amount of from 1 to 10% by weight of the solid hair colouring composition. In one aspect the water soluble hair colouring is present in an amount of from 0.1 to 5% by weight of the solid hair colouring composition. In one aspect the water soluble hair colouring is present in an amount of from 1 to 5% by weight of the solid hair colouring composition.

### Vegetable Oil, Vegetable Butter, Wax Or Mixtures Thereof

The solid hair colouring composition of the present invention may contain vegetable oil, vegetable butter, wax or mixture thereof. In one aspect the solid hair colouring composition contains vegetable oil. In one aspect the solid hair colouring composition contains vegetable butter. In one aspect the solid hair colouring composition contains vegetable oil and vegetable butter. The vegetable oil, vegetable butter, wax or mixture thereof may be present in any suitable amount to provide the necessary properties of the final product. In one preferred aspect the vegetable oil, vegetable butter and wax are present in a total amount of from 5 to 50% by weight of the solid hair colouring composition, such as in a total amount of from 3 to 35% by weight of the solid hair colouring composition, such as in a total amount of from 10 to 40% by weight of the solid hair colouring composition, such as in an amount of from 15 to 40% by weight of the solid hair colouring composition, such as in an amount of from 15 to 35% by weight of the solid hair colouring composition, such as in a total amount of from 10 to 25% by weight of the solid hair colouring composition,.

It will be understood by one skilled in the art that the wax referred to in this section is in addition to any emulsifying wax of the hair conditioner concentrate. Therefore in one aspect the solid hair colouring composition of the present invention comprises a wax and a hair conditioner concentrate comprising an emulsifying wax wherein the wax and the emulsifying wax are different.

The vegetable oil, vegetable butter and wax may be selected from any materials suitable to achieve the purpose of the present invention. In one preferred aspect, the vegetable oil, vegetable butter and wax are selected from olive oil, jojoba oil, almond oil, avocado oil, lanolin, castor oil, moringa oil, sesame oil, rosehip oil, raspberry seed oil, shea butter, cocoa butter, murumuru butter, cupuacu butter, illipe butter, mango butter, beeswax, rapeseed wax, japan wax and mixtures thereof. In one preferred aspect, the vegetable oil, vegetable butter and wax are selected from jojoba oil, almond oil, avocado oil, lanolin, castor oil, sesame oil, rosehip oil, raspberry seed oil, shea butter, cocoa butter, murumuru butter, cupuacu butter, illipe butter, mango butter, beeswax, rapeseed wax, japan wax and mixtures thereof. In one preferred aspect, the vegetable oil, vegetable butter and wax are selected from olive oil, jojoba oil, almond oil, avocado oil, lanolin, castor oil, cocoa butter and shea butter and mixtures thereof. In one preferred aspect, the vegetable oil, vegetable butter and wax are selected from jojoba oil, almond oil, avocado oil, lanolin, castor oil, cocoa butter and shea butter and mixtures thereof.

In one preferred aspect the vegetable oil, vegetable butter, wax or mixture thereof is vegetable oil. In one preferred aspect the vegetable oil is selected from jojoba oil, almond oil, avocado oil, castor oil, moringa oil, olive oil, sesame oil, rosehip oil, raspberry seed oil, and mixtures thereof. In one aspect the vegetable oil is selected from jojoba oil, almond oil, avocado oil, castor oil, moringa oil, olive oil and mixtures thereof. In one preferred aspect the vegetable oil is selected from olive oil, jojoba oil and mixtures thereof. Olive oil is particularly favourable for use within the present invention. Olive oil is rich in squalene, which is also found in sebum that coats the skin and hair. The compatible nature of sebum and olive oil results in the rapid absorption of olive oil onto the hair fibres, improving the lipid levels of the cell membrane cortex, thus protecting the overall health of the cuticle and cortex, without causing an undesirable greasy sensation on the hair.

The oils, butters and waxes that may be incorporated into the composition may be selected by one skilled in the art based on their suitability and the type of hair to be treated with the hair conditioner. Some details of the oils and the hair types which they are used are as follows:
Olive oil - used in all the formulation as it is the only oil proven to improve the elastic strength of the hair. It helps the cuticle and the cortex of the hair stretch.
Jojoba oil - used as it is a liquid wax, resulting in a dry finish when used on the hair and tends to improve the condition of dry and fine hair.
Almond Oil - softens and adds shine to the hair. Suitable for all hair types.
Avocado Oil - adds strength and shine to the hair. Suitable for all hair types.
Lanolin - resembles the hair's sebum. It is an excellent emollient and an effective moisturiser. It coats and protects the hair, making it a very effective conditioner of dry hair.
Castor Oil - rich oil, to protect the hair but also adds strength and shine. Suitable for dry and damaged hair.

### Hydrocolloid

The solid hair colouring composition of the present invention further comprises at least one hydrocolloid. The hydrocolloid may be present in any suitable amount to provide the necessary properties of the final product. The solid hair colouring composition may contain hydrocolloid in an amount of at least 3 % by weight of the solid hair colouring composition. The solid hair colouring composition may contain hydrocolloid in an amount of at least 5 % by weight of the solid hair colouring composition. The solid hair colouring composition may contain hydrocolloid in an amount of no greater than 20 % by weight of the solid hair colouring composition. The solid hair colouring composition may contain hydrocolloid in an amount of no greater than 15 % by weight of the solid hair colouring composition. The solid hair colouring composition may contain hydrocolloid in an amount of no greater than 10 % by weight of the solid hair colouring composition.

In one aspect the hydrocolloid is present in an amount of from 5 to 20% by weight of the solid hair colouring composition. In one aspect the hydrocolloid is present in an amount of from 5 to 10% by weight of the solid hair colouring composition.

The hydrocolloid is selected from cationic guar gum, behenyl trimethyl ammonium chloride, polyquaternium-81, cetrimonium bromide, and mixtures thereof. In one preferred aspect the hydrocolloid is cationic guar gum.

In the present invention the hydrocolloid is able to act as a thickening and structural component. This facilitates the application of the hydrated cosmetic composition to the user's hair, allowing for the composition to remain on the hair fibres and the dye to diffuse across the hair's cuticle and deposit within the cortex. This enhances the effective colouring of a user's hair, as the composition should remain on the hair fibres for a sufficient amount of time, so that adequate diffusion and deposition of the dye can occur to cause a noticeable change in the appearance of a user's hair. It has been found that without the hydrocolloid the composition has sub-optimal structural integrity on the hair, thereby resulting in the reduction of the dye's ability to transcend across the hair's cuticle and deposit within the cortex.

In one preferred aspect the hydrocolloid is cationic guar gum. Guar gum refers to the mucilage present in the seed of the *Cyamopsis tetragonoloba* plant, and is widely known for its industrial applications as a thickening, stabilising and dietary additive. The use of guar gum in the present invention may be advantageous as it produces a viscous pseudoplastic solution that enables the effective suspension of active ingredients and other additives when dissolved and dispersed within water. Furthermore, due to the inherent low-shear viscosity of guar gum solutions, the composition of the present invention can be readily applied to the hair, coating the individual hair shafts and allowing the active ingredients to condition the cuticle, without any loss of structural cohesion or integrity of the composition on the hair.

A preferred solid hair colouring composition of the present invention comprises
(i) a hair conditioner concentrate in an amount of from 30 to 65% by weight of the solid hair colouring composition, wherein the hair conditioner concentrate is a combination of cetearyl alcohol and sodium lauryl sulphate (SLS) present in a ratio of from 20:1 to 2:1 by weight.
(ii) a water soluble hair colouring present in an amount of from 0.1 to 5% by weight of the solid hair colouring composition;
(iii) jojoba oil, almond oil, avocado oil, castor oil, moringa oil, olive oil, lanolin or a mixture thereof, present in a total amount of from 15 to 40% by weight of the solid hair colouring composition.
(iv) at least one hydrocolloid selected from cationic guar gum, behenyl trimethyl ammonium chloride, polyquaternium-81, cetrimonium bromide and mixtures thereof, wherein the hydrocolloid is present in an amount of from 5 to 20% by weight of the solid hair colouring composition.

A further solid hair colouring composition of the present invention comprises
(i) a hair conditioner concentrate in an amount of from 50 to 65% by weight of the solid hair colouring composition, wherein the hair conditioner concentrate is a combination of cetearyl alcohol and sodium lauryl sulphate (SLS) present in a ratio of from 20:1 to 2:1 by weight.
(ii) a water soluble hair colouring present in an amount of from 0.1 to 5% by weight of the solid hair colouring composition;
(iii) jojoba oil, almond oil, avocado oil, castor oil, moringa oil, olive oil, lanolin or a mixture thereof, present in a total amount of from 15 to 30% by weight of the solid hair colouring composition.
(iv) at least one hydrocolloid selected from cationic guar gum, behenyl trimethyl ammonium chloride, polyquaternium-81, cetrimonium bromide and mixtures thereof, wherein the hydrocolloid is present in an amount of from 5 to 20% by weight of the solid hair colouring composition.

### Acidifying Agent

The solid hair colouring composition of the present invention urther comprises at least one acidifying agent. The acidifying agent is present in an amount of from 3 to 25% by weight of the solid hair colouring composition.

In one aspect the acidifying agent is present in an amount of from 5 to 25% by weight of the solid hair colouring composition. In one aspect the acidifying agent is present in an amount of from 6 to 20% by weight of the solid hair colouring composition. In one aspect the acidifying agent is present in an amount of from 6 to 15% by weight of the solid hair colouring composition. In one aspect the acidifying agent is present in an amount of from 6 to 10% by weight of the solid hair colouring composition.

The acidifying agent may be selected from all suitable acidifying agents. In one aspect the acidifying agent is selected from organic acids. In one aspect the acidifying agent is selected from monocarboxylic acids, dicarboxylic acids, tricarboxylic acids and mixtures thereof. In one aspect the acidifying agent is selected citric acid, tartaric acid, potassium bitartrate (cream of tartar) and mixtures thereof. In one aspect the acidifying agent is citric acid. In one aspect the acidifying agent is cream of tartar. In one aspect the acidifying agent is a mixture of citric acid and cream of tartar.

It has been particularly been found that by incorporating an organic acid into a composition according to the present invention, the isoelectric point of the cuticle proteins could be achieved, negating the potential risk of adverse ionic interactions occurring between the dye and the cuticle proteins, preventing the diffusion of a dye across the hair's cuticle.

In one aspect the solid hair colouring composition of the present invention further comprises at least one acidifying agent (such as citric acid and/or cream of tartar) and at least one hydrocolloid (such as cationic guar gum). As discussed above we have by providing a hair conditioner concentrate (such as an emulsifying wax) and applying this to the hair in combination with water and the heat from the warm water, the hair fibres are caused to swell and the cuticles to raise. This increases the ability of the dye to pass across the cuticle membrane and deposit within the cortex of the hair. After a while the hydrocolloid and acidifying agent cause the cuticles to lay flat, smoothing the appearance of the hair fibres and sealing the dye within the hair fibre. Therefore the dual combined effect of the components of the composition improves the ability of the dye to permeate into the cortex and further aids the retention of the dye within said cortex and improves the finished appearance of the user's coloured hair.

### Unit Dose

As discussed herein, we have found that by providing the present system a user may make a freshly prepared batch of hair colouring. This may be done with minimal experience and creating minimal mess. To further simplify the preparation of the product, in one aspect the composition is provided as a unit dose. To prepare the colouring, this unit dose may be combined with the necessary amount of water. Yet further we have found that the easy preparation may be improved by embedding a stirring device in the unit dose of solid hair colouring. By this means, the solid hair colouring composition may be placed in water and as it begins to disperse the stirring device may be used to agitate the water and further disperse the solid hair colouring. Once the solid hair colouring has completely disintegrated such that the stirrer is no longer embedded within it, the stirrer may be used to further mix the dispersed hair colouring. The provision of a unit dose is also advantageous because it allows for the preparation of enough hair colouring for one application so waste product is not created.

Therefore, in a further aspect there is provided a unit dose of a solid cosmetic composition, wherein the unit dose comprises
(a) a solid hair colouring composition in a predetermined amount
(b) a stirring device for stirring a liquid
wherein the stirring device is partially embedded within the solid hair colouring composition.

The solid hair colouring composition is a solid hair colouring composition as defined hereinabove.

To further simplify the preparation of the composition, preferably the unit dose denotes the amount of liquid into which the predetermined amount of solid hair colouring composition should be dispersed in use. This may be simply done by marking the amount of liquid on the stirrer. For example the stirrer may be a wooden implement such as a spoon onto which is marked the amount of liquid into which the solid unit dose is to be combined.

The stirring device may be any suitable stirrer such as a spatula, fork, spoon or other utensil. The stirring device may be made from any suitable material such wood, metal, plastic or combinations thereof.

### Further Components

The solid hair colouring composition may contain one or more additional components such as to provide the desired composition. In one aspect the solid hair colouring composition further comprising at least one additional component selected from salt, sugars, nuts, flours, humectants, surfactants, fruits, vegetables, herbs, seaweeds, cereals, beans, proteins, binders, fillers, dispersants, opacifiers, perfumes, colours, fragrances, sunscreens and mixtures thereof. In one aspect the solid hair colouring composition further comprising at least one additional component selected from sugars, nuts, flours, humectants, surfactants, fruits, vegetables, herbs, seaweeds, cereals, beans, proteins, binders, fillers, dispersants, opacifiers, perfumes, colours, fragrances, and mixtures thereof. In one aspect, the solid hair colouring composition comprising a further component selected from salt, binders, fillers, fruits, vegetables, humectants, dispersants, surfactants, sunscreens and mixtures thereof. In one aspect, the solid hair colouring composition comprising a further component selected from binders, fillers, fruits, vegetables, humectants, dispersants, and mixtures thereof.

In one aspect the solid hair colouring composition contains one or more fragrances. Preferably the solid hair colouring composition comprises fragrance in an amount of no greater than 6 % by weight of the colouring composition. Preferably the solid hair colouring composition comprises fragrance in an amount of no greater than 5 % by weight of the colouring composition. If present, fragrance may be present in an amount of from 0.01 to 6% by weight of the total composition. If present, fragrance may be present in an amount of from 0.01 to 5% by weight of the total composition. The amount of fragrances is preferably from 0.1% to 5% by weight of the total composition, such as from 0.1% to 4% by weight of the total composition, such as from 0.5% to 5% by weight of the total composition, such as from 1% to 5% by weight of the total composition, such as from 0.5% to 4% by weight of the total composition, such as from 0.5% to 3% by weight of the total composition, such as from 0.5% to 2.5% by weight of the total composition, such as from 1.5% to 2.5% by weight of the total composition. Alternatively, in one aspect, the solid hair colouring composition is fragrance free.

The essential oils may be selected based on the fragrance desired, skin type to be treated and other effects desired based on the well-known properties of essential oils. The addition of essential oils, when taken in to the nose, is known to alter mood. For example, essential oils are known to create effects of drowsiness or stimulating the senses. Many effects can be achieved by the use of essential oils.

In one embodiment, the one or more essential oils present in the product are selected from Rosewood, Sandalwood, Chamomile, Eucalyptus, Tonka absolute, Lemon myrtle, Jasmin, Ylang ylang, Labdanum, Lemongrass, Rose Absolute, Grapefruit, Patchouli, Rosemary, Armois, Lemon, Neroli, Sweet violet, Lavender, Orange 50 fold, Vanilla, Peppermint, Benzoin, Hydrangia, Litsea Cubeba, Cardamon, Tonka, and Chamomile blue. In one embodiment, the one or more essential oils present in the product are selected from Rosewood, Sandalwood, Chamomile, Eucalyptus, Lavender, Tonka absolute, Rose absolute.

Vitamins, particularly B, C and E are very beneficial for the hair and scalp. Vitamin rich ingredients such as Wheatgerm oil can also be used to deliver vitamins on to the hair and scalp. In a one embodiment, the vitamins are selected from vitamin B, vitamin C, vitamin E and mixtures thereof. It will be appreciated by one skilled in the art that the vitamin may be provided from any suitable source. For example the vitamin(s) may be provided from a synthetic source or from incorporation into the product of a material, such as a natural material, that has a high vitamin content.

In one aspect the solid hair colouring composition contains a humectant. In one aspect, the humectant is selected from honey, glycerine, sorbitol, agave nectar, fruit syrups, herbal syrups and mixtures thereof. Preferably the humectant is selected from honey, glycerine, sorbitol and mixtures thereof.

In one aspect the solid hair colouring composition comprises a sunscreen. In one aspect the sunscreen is a physical sunscreen (UV reflecting material). In one aspect the sunscreen is a chemical sunscreen (UV absorbing material). In one preferred aspect the sunscreen is a UV absorbing material is a material that absorbs at least UVA or UVB radiation. In one preferred aspect the sunscreen is a UV absorbing material selected from any materials suitable to achieve the absorption of UV radiation. In some embodiments, the sunscreen is a UV absorbing material selected from sulisobenzone, a salt of sulisobenzone, oxybenzone, octocrylene, octyl methoxycinnamate, butylmethoxydibenzoylmethane, homosalate, ecamsule, and mixtures thereof. In one preferred aspect the sunscreen is selected from sulisobenzone, the sodium salt of sulisobenzone, oxybenzone and mixtures thereof. As the skilled person will appreciate, oxybenzone is commonly referred to as benzophenone-3, sulisobenzone is commonly referred to as benzophenone-4 and the sodium salt of sulisobenzone is commonly referred to as benzophenone-5. In one aspect the solid hair colouring composition comprises a sunscreen (such as benzophenone-3, benzophenone-4 and/or benzophenone-5) in an amount of from 0.1 to 10% by weight of the solid hair colouring composition, such as from 0.5 to 5% by weight of the solid hair colouring composition.

In one aspect the solid hair colouring composition comprises a surfactant. In one aspect the solid hair colouring composition comprises a surfactant selected from sodium lauryl sulfate, sodium laureth sulfate, sodium lauryl sulfoacetate, ammonium lauryl sulfate, sodium laureth sulfate, sodium cocoamphoacetate, disodium laureth sulfosuccinate, lauryl betaine and mixtures thereof. In one aspect the solid hair colouring composition comprises a surfactant in an amount of from 0.1 to 10% by weight of the solid hair colouring composition, such as in an amount of from 1 to 9% by weight of the solid hair colouring composition, such as in an amount of from 5 to 8% by weight of the solid hair colouring composition.

In one aspect the solid hair colouring composition contains water. In one aspect the solid hair colouring composition is free or substantially free of water. Preferably the solid hair conditioner composition comprises water in an amount of no greater than 5 % by weight of the solid cosmetic composition, such as in an amount of no greater than 4 % by weight of the solid cosmetic composition, such as in an amount of no greater than 3 % by weight of the solid cosmetic composition, such as in an amount of no greater than 2 % by weight of the solid cosmetic composition, such as in an amount of no greater than 1 % by weight of the solid cosmetic composition, such as in an amount of no greater than 0.5 % by weight of the solid cosmetic composition, such as in an amount of no greater than 0.1 % by weight of the solid cosmetic composition, such as in an amount of no greater than 0.01 % by weight of the solid cosmetic composition.

In one aspect the solid hair colouring composition contains preservatives. In one aspect the solid hair colouring composition is free or substantially free of preservatives. Preferably the solid hair conditioner composition comprises preservatives in an amount of no greater than 5 % by weight of the solid cosmetic composition, such as in an amount of no greater than 4 % by weight of the solid cosmetic composition, such as in an amount of no greater than 3 % by weight of the solid cosmetic composition, such as in an amount of no greater than 2 % by weight of the solid cosmetic composition, such as in an amount of no greater than 1 % by weight of the solid cosmetic composition, such as in an amount of no greater than 0.5 % by weight of the solid cosmetic composition, such as in an amount of no greater than 0.1 % by weight of the solid cosmetic composition, such as in an amount of no greater than 0.01 % by weight of the solid cosmetic composition.

An advantage of the solid hair colouring composition of the present invention is that it does not require packaging. Thus preferably the solid hair colouring composition is provided free of packaging. It will be understood that the product may include labelling providing information on the composition of the product and/or its usage. The primary purpose of such labelling is to carry information rather than to pack the product.

The above ranges provide preferred amounts of each of the components. Each of these ranges may be taken alone or in combination with one or more other component ranges to provide a preferred aspect of the invention.

### Use

To use the product of the present invention, the end user will typically place the product in a container. A small amount of warm or hot water can then be added to the container, typically to just cover the solid product. The product will start to disperse. Once a significant amount of product has dispersed more water can be added and the product gently stirred. Water can then be added until the desired consistency of dispersed product has been formed. This consistency may be determined by the user based on, for example, their preference or their hair type or condition.

In one aspect the product is dispersed in a predetermined amount of water. In one aspect the unit dose of the present invention is dispersed in from 200 to 750ml of water, such as from 200 to 500ml of water, such as from 250 to 500ml of water, such as approximately 250ml of water.

### Example

The invention will now be described with reference to the following non-limiting example.

| **Batch Size:** | | **(g)** |
|---|---|---|
| **Formula %** | **Raw Material Type** | |
| | | |
| 61.00 | Cetearyl Alcohol & SLS | 1220 |
| 2.00 | CI Natural red 3 | 40 |
| 25.00 | Olive Oil | 500 |
| 6.00 | Citric Acid | 120 |
| 6.00 | Guar gum | 120 |
| | | |
| 100.00 | | 2000 |

### Production Method

1. The cetearyl alcohol and sodium lauryl sulphate (SLS) are warmed to a temperature in the range of 60°C - 90°C, with 65°C being the ideal temperature until completely molten.
2. The oils are then added to the cetearyl alcohol and SLS when the temperature falls within the range of 45°C - 70°C, with 50°C being the ideal temperature.
3. The guar gum is then stirred through the oil phase, once a temperature of between 35°C - 55°C has been reached, with the ideal temperature being 45°C.
4. When the temperature falls below 35°C, the citric acid and water-soluble colourant can be added and thoroughly incorporated within the composition, along with any fragrance material or other desired additives.
5. The product is then poured into moulds, and a stirring device is added and cooled to between 4°C - 10°C to set.
6. Once set the product is removed is ready to use.

Although the invention has been described in connection with specific preferred embodiments, it should be understood that the invention as claimed should not be unduly limited to such specific embodiments. Indeed, various modifications of the described modes for carrying out the invention which are obvious to those skilled in chemistry, biology or related fields are intended to be within the scope of the following claims.

## Claims

1. A solid hair colouring composition comprising
(i) a hair conditioner concentrate selected from emulsifying waxes, sodium polynaphthalene sulfonate, stearamidopropyl dimethylamine (aka N-[3-(Dimethylamino)propyl] octadecanamide) and mixtures thereof;
(ii) a water soluble hair colouring having a solubility of more than 20 g/L water at 25°C;
(iii) an acidifying agent in an amount of from 3% to 25% by weight of the solid hair colouring composition; and
(iv) a hydrocolloid, wherein the hydrocolloid is selected from cationic guar gum, behenyl trimethyl ammonium chloride, polyquaternium-81, cetrimonium bromide and mixtures thereof;
wherein the solid hair colouring composition can substantially sustain its physical shape when unsupported by external means, and remains substantially solid at up to 30°C.

2. A solid hair colouring composition according to claim 1, wherein the hair conditioner concentrate is present in an amount of at least 20 % by weight of the solid hair colouring composition.

3. A solid hair colouring composition according to claim 1 or 2, wherein the hair conditioner concentrate is selected from emulsifying waxes, preferably wherein the emulsifying wax is a combination of cetearyl alcohol and sodium lauryl sulphate (SLS).

4. A solid hair colouring composition according to any one of the preceding claims, wherein the water soluble hair colouring is present in an amount of from 0.01 to 10% by weight of the solid hair colouring composition.

5. A solid hair colouring composition according to any one of the preceding claims, wherein the water soluble hair colouring is selected from chlorophyll, carotenoids, anthocyanins, betalains and mixtures thereof.

6. A solid hair colouring composition according to any one of the preceding claims, wherein the water soluble hair colouring is a non-oxidative dye.

7. A solid hair colouring composition according to any one of the preceding claims further comprising vegetable oil, vegetable butter, wax or a mixture thereof.

8. A solid hair colouring composition according to claim 7 wherein vegetable oil, vegetable butter and wax are present in a total amount of from 5 to 35% by weight of the solid hair colouring composition.

9. A solid hair colouring composition according to claim 7 or 8, wherein the vegetable oil, vegetable butter, wax or mixture thereof is vegetable oil, preferably wherein the vegetable oil is selected from jojoba oil, almond oil, avocado oil, castor oil, moringa oil, olive oil and mixtures thereof.

10. A solid hair colouring composition according to any one of the preceding claims, wherein the hydrocolloid is present in an amount of from 5 to 20% by weight of the solid hair colouring composition.

11. A solid hair colouring composition according to any one of the preceding claims, wherein the acidifying agent is citric acid.

12. A solid hair colouring composition according to any one of the preceding claims, wherein the acidifying agent is present in an amount of from 5 to 15% by weight of the solid hair colouring composition.

13. A solid hair colouring composition according to claim 1 comprising
(i) a hair conditioner concentrate in an amount of from 50 to 65% by weight of the solid hair colouring composition, wherein the hair conditioner concentrate is a combination of cetearyl alcohol and sodium lauryl sulphate (SLS) present in a ratio of from 20:1 to 2:1 by weight.
(ii) a water soluble hair colouring present in an amount of from 0.1 to 5% by weight of the solid hair colouring composition;
(iii) vegetable oil, vegetable butter, wax or a mixture thereof present in a total amount of from 15 to 30% by weight of the solid hair colouring composition, wherein the vegetable oil is selected from jojoba oil, almond oil, avocado oil, castor oil, moringa oil, olive oil, or a mixture thereof;
(iv) at least one hydrocolloid selected from cationic guar gum, behenyl trimethyl ammonium chloride, polyquaternium-81, cetrimonium bromide and mixtures thereof, wherein the hydrocolloid is present in an amount of from 5 to 20% by weight of the solid hair colouring composition; and
(v) an acidifying agent in an amount of from 5% to 15% by weight of the solid hair conditioning composition.

14. A unit dose of a solid cosmetic composition, wherein the unit dose comprises
(a) a solid hair colouring composition as defined in any one of claims 1 to 13 in a predetermined amount; and
(b) a stirring device for stirring a liquid,
wherein the stirring device is partially embedded within the solid hair colouring composition.

15. A method of preparing a hair colouring composition comprising the step of contacting water and a solid hair colouring composition as defined in claim 1.

## Patentansprüche

1. Feste Haarfärbezusammensetzung, umfassend
(i) ein Haarkonditionierungsmittelkonzentrat, ausgewählt aus emulgierenden Wachsen, Natriumpolynaphthalinsulfonat, Stearamidopropyldimethylamin (auch bekannt als N-[3-(Dimethylamino)propyl]octadecanamid) und Mischungen davon;
(ii) ein wasserlösliches Haarfärbemittel mit einer Löslichkeit von mehr als 20 g/l Wasser bei 25 °C;
(iii) ein Ansäuerungsmittel in einer Menge von 3 bis 25 Gew.-%, bezogen auf die feste Haarfärbezusammensetzung; und
(iv) ein Hydrokolloid, wobei das Hydrokolloid aus kationischem Guargummi, Behenyltrimethylammoniumchlorid, Polyquaternium-81, Cetrimoniumbromid und Mischungen davon ausgewählt ist;
wobei die feste Haarfärbezusammensetzung ihre physikalische Form ohne Stützung durch externe Mittel weitgehend beibehalten kann und bei bis zu 30 °C weitgehend fest bleibt.

2. Feste Haarfärbezusammensetzung nach Anspruch 1, wobei das Haarkonditionierungsmittelkonzentrat in einer Menge von mindestens 20 Gew.-%, bezogen auf die feste Haarfärbezusammensetzung, vorliegt.

3. Feste Haarfärbezusammensetzung nach Anspruch 1 oder 2, wobei das Haarkonditionierungsmittelkonzentrat aus emulgierenden Wachsen ausgewählt ist, vorzugsweise wobei es sich bei dem emulgierenden Wachs um eine Kombination von Cetearylalkohol und Natriumlaurylsulfat (SLS) handelt.

4. Feste Haarfärbezusammensetzung nach einem der vorhergehenden Ansprüche, wobei das wasserlösliche Haarfärbemittel in einer Menge von 0,01 bis 10 Gew.-%, bezogen auf die feste Haarfärbezusammensetzung, vorliegt.

5. Feste Haarfärbezusammensetzung nach einem der vorhergehenden Ansprüche, wobei das wasserlösliche Haarfärbemittel aus Chlorophyll, Carotinoiden, Anthocyaninen, Betalainen und Mischungen davon ausgewählt ist.

6. Feste Haarfärbezusammensetzung nach einem der vorhergehenden Ansprüche, wobei es sich bei dem wasserlöslichen Haarfärbemittel um einen nichtoxidativen Farbstoff handelt.

7. Feste Haarfärbezusammensetzung nach einem der vorhergehenden Ansprüche, ferner umfassend pflanzliches Öl, pflanzliche Butter, Wachs oder eine Mischung davon.

8. Feste Haarfärbezusammensetzung nach Anspruch 7, wobei pflanzliches Öl, pflanzliche Butter und Wachs in einer Gesamtmenge von 5 bis 35 Gew.-%, bezogen auf die feste Haarfärbezusammensetzung, vorliegen.

9. Feste Haarfärbezusammensetzung nach Anspruch 7 oder 8, wobei es sich bei dem pflanzlichen Öl, der pflanzlichen Butter, dem Wachs oder der Mischung davon um pflanzliches Öl handelt, vorzugsweise wobei das pflanzliche Öl aus Jojobaöl, Mandelöl, Avocadoöl, Rizinusöl, Moringaöl, Olivenöl und Mischungen davon ausgewählt ist.

10. Feste Haarfärbezusammensetzung nach einem der vorhergehenden Ansprüche, wobei das Hydrokolloid in einer Menge von 5 bis 20 Gew.-%, bezogen auf die feste Haarfärbezusammensetzung, vorliegt.

11. Feste Haarfärbezusammensetzung nach einem der vorhergehenden Ansprüche, wobei es sich bei dem Ansäuerungsmittel um Citronensäure handelt.

12. Feste Haarfärbezusammensetzung nach einem der vorhergehenden Ansprüche, wobei das Ansäuerungsmittel in einer Menge von 5 bis 15 Gew.-%, bezogen auf die feste Haarfärbezusammensetzung, vorliegt.

13. Feste Haarfärbezusammensetzung nach Anspruch 1, umfassend
(i) ein Haarkonditionierungsmittelkonzentrat in einer Menge von 50 bis 65 Gew.-%, bezogen auf die feste Haarfärbezusammensetzung, wobei es sich bei dem Haarkonditionierungsmittelkonzentrat um eine Kombination von Cetearylalkohol und Natriumlaurylsulfat (SLS) in einem Gewichtsverhältnis von 20:1 bis 2:1 handelt.
(ii) ein wasserlösliches Haarfärbemittel in einer Menge von 0,1 bis 5 Gew.-%, bezogen auf die feste Haarfärbezusammensetzung; und
(iii) pflanzliches Öl, pflanzliche Butter, Wachs oder eine Mischung davon in einer Gesamtmenge von 15 bis 30 Gew.-%, bezogen auf die feste Haarfärbezusammensetzung, wobei das pflanzliche Öl aus Jojobaöl, Mandelöl, Avocadoöl, Rizinusöl, Moringaöl, Olivenöl und einer Mischung davon ausgewählt ist;
(iv) mindestens ein Hydrokolloid, das aus kationischem Guargummi, Behenyltrimethylammoniumchlorid, Polyquaternium-81, Cetrimoniumbromid und Mischungen davon ausgewählt ist, wobei das Hydrokolloid in einer Menge von 5 bis 20 Gew.-%, bezogen auf die feste Haarfärbezusammensetzung, vorliegt; und
(v) ein Ansäuerungsmittel in einer Menge von 5 bis 15 Gew.-%, bezogen auf die feste Haarkonditionierungsmittelzusammensetzung.

14. Einzeldosis einer festen kosmetischen Zusammensetzung, wobei die Einzeldosis Folgendes umfasst:
(a) eine feste Haarfärbezusammensetzung gemäß einem der Ansprüche 1 bis 13 in einer vorbestimmten Menge; und
(b) eine Rührvorrichtung zum Rühren einer Flüssigkeit, wobei die Rührvorrichtung teilweise in die feste Haarfärbezusammensetzung eingebettet ist.

15. Verfahren zur Herstellung einer Haarfärbezusammensetzung, umfassend den Schritt des Inkontaktbringens von Wasser und einer festen Haarfärbezusammensetzung gemäß Anspruch 1.

## Revendications

1. Composition de coloration capillaire solide comprenant
(i) un concentré d'après-shampooing choisi parmi des cires émulsifiantes, le sodium polynaphthalene sulfonate, le stearamidopropyl dimethylamine (c'est-à-dire N-[3-(Diméthylamino)propyl]octadécanamide) et des mélanges correspondants ;
(ii) une matière colorante capillaire soluble dans l'eau ayant une solubilité supérieure à 20 g/L d'eau à 25 °C ;
(iii) un agent acidifiant en une quantité allant de 3 % à 25 % en poids de la composition de coloration capillaire solide ; et
(iv) un hydrocolloïde, l'hydrocolloïde étant choisi parmi une gomme de guar cationique, le chlorure de béhényltriméthylammonium, le polyquaternium-81, le bromure de cétrimonium et des mélanges correspondants ;
la composition de coloration capillaire solide pouvant maintenir sensiblement sa forme physique lorsqu'elle est non supportée par un moyen externe, et restant sensiblement solide jusqu'à 30 °C.

2. Composition de coloration capillaire solide selon la revendication 1, le concentré d'après-shampooing étant présent en une quantité d'au moins 20 % en poids de la composition de coloration capillaire solide.

3. Composition de coloration capillaire solide selon la revendication 1 ou 2, le concentré d'après-shampooing étant choisi parmi des cires émulsifiantes, préférablement la cire émulsifiante étant une combinaison d'alcool cétéarylique et de laurylsulfate de sodium (SLS) .

4. Composition de coloration capillaire solide selon l'une quelconque des revendications précédentes, la matière colorante capillaire soluble dans l'eau étant présente en une quantité allant de 0,01 à 10 % en poids de la composition de coloration capillaire solide.

5. Composition de coloration capillaire solide selon l'une quelconque des revendications précédentes, la matière colorante capillaire soluble dans l'eau étant choisie parmi la chlorophylle, des caroténoïdes, des anthocyanines, des betalaïnes et des mélanges correspondants.

6. Composition de coloration capillaire solide selon l'une quelconque des revendications précédentes, la matière colorante capillaire soluble dans l'eau étant un colorant non oxydant.

7. Composition de coloration capillaire solide selon l'une quelconque des revendications précédentes, comprenant en outre une huile végétale, un beurre végétal, une cire ou un mélange correspondant.

8. Composition de coloration capillaire solide selon la revendication 7, une huile végétale, un beurre végétal et une cire étant présent(e)s en une quantité totale allant de 5 à 35 % en poids de la composition de coloration capillaire solide.

9. Composition de coloration capillaire solide selon la revendication 7 ou 8, l'huile végétale, le beurre végétal, la cire ou un mélange correspondant étant une huile végétale, préférablement l'huile végétale étant choisie parmi l'huile de jojoba, l'huile d'amande, l'huile d'avocat, l'huile de ricin, l'huile de moringa, l'huile d'olive et des mélanges correspondants.

10. Composition de coloration capillaire solide selon l'une quelconque des revendications précédentes, l'hydrocolloïde étant présent en une quantité allant de 5 à 20 % en poids de la composition de coloration capillaire solide.

11. Composition de coloration capillaire solide selon l'une quelconque des revendications précédentes, l'agent acidifiant étant l'acide citrique.

12. Composition de coloration capillaire solide selon l'une quelconque des revendications précédentes, l'agent acidifiant étant présent en une quantité allant de 5 à 15 % en poids de la composition de coloration capillaire solide.

13. Composition de coloration capillaire solide selon la revendication 1 comprenant
(i) un concentré d'après-shampooing en une quantité allant de 50 à 65 % en poids de la composition de coloration capillaire solide, le concentré d'après-shampooing étant une combinaison d'alcool cétéarylique et de laurylsulfate de sodium (SLS) présents en un rapport allant de 20:1 à 2:1 en poids ;
(ii) une matière colorante capillaire soluble dans l'eau en une quantité allant de 0,1 à 5 % en poids de la composition de coloration capillaire solide ;
(iii) une huile végétale, un beurre végétal, une cire ou un mélange correspondant présent(e)s en une quantité totale allant de 15 à 30 % en poids de la composition de coloration capillaire solide, l'huile végétale étant choisie parmi l'huile de jojoba, l'huile d'amande, l'huile d'avocat, l'huile de ricin, l'huile de moringa, l'huile d'olive et un mélange correspondant ;
(iv) au moins un hydrocolloïde choisi parmi une gomme de guar cationique, le chlorure de béhényltriméthylammonium, le polyquaternium-81, le bromure de cétrimonium et des mélanges correspondants, l'hydrocolloïde étant présent en une quantité allant de 5 à 20 % en poids de la composition de coloration capillaire solide ; et
(v) un agent acidifiant en une quantité allant de 5 % à 15 % en poids de la composition d'après-shampooing solide.

14. Dose unitaire d'une composition cosmétique solide, la dose unitaire comprenant
(a) une composition de coloration capillaire solide telle que définie dans l'une quelconque des revendications 1 à 13 en une quantité prédéterminée ; et
(b) un dispositif d'agitation pour l'agitation d'un liquide,
le dispositif d'agitation étant partiellement incorporé dans la composition de coloration capillaire solide.

15. Procédé de préparation d'une composition de coloration capillaire comprenant l'étape de mise en contact d'eau et d'une composition de coloration capillaire solide telle que défini dans la revendication 1.
